# EUROPEAN PATENT SPECIFICATION

(11) **EP 1 478 414 B1**
(45) Date of publication and mention of the grant of the patent: **27.12.2006**
(21) Application number: 03717892.8
(22) Date of filing: 13.02.2003
(51) Int. Cl.: A61L 27/40

(54) **METAL REINFORCED BIODEGRADABLE INTRALUMINAL STENTS**
METALLVERSTÄRKTER BIO-ABBAUBARER INTRALUMINALER STENT
EXTENSEURS ENDOLUMINAUX BIODEGRADABLES A RENFORT METALLIQUE

(30) Priority: 14.02.2002 US 75914
(43) Date of publication of application: 24.11.2004
(73) Proprietor: Boston Scientific Limited, St. Michael (BB)
(72) Inventor: CHANDRASEKARAN, Chandru, Mercer Island, WA 98040 (US)
(74) Representative: Schildberg, Peter
(86) International application number: PCT/US2003/004442
(87) International publication number: WO 2003/068285

(56) References cited:
- EP-A- 1 110 561
- WO-A-01/35859
- WO-A-01/49335
- US-A- 5 443 496
- US-A- 5 674 241

## Description

### FIELD OF THE INVENTION

The present invention relates to implantable or insertable medical devices, particularly to intraluminal stents constructed of a composite of metallic and biodegradable materials.

### BACKGROUND OF THE INVENTION

Intraluminal stents are typically inserted or implanted into a body lumen, for example, a coronary artery, after a procedure such as percutaneous transluminal coronary angioplasty ("PCTA"). Such stents are used to maintain the patency of the coronary artery by supporting the arterial walls and preventing abrupt reclosure or collapse thereof which can occur after PCTA. These stents can also be provided with one or more therapeutic agents adapted to be locally released from the stent at the site of implantation. In the case of a coronary stent, the stent can be adapted to provide release of, for example, an antithrombotic agent to inhibit clotting or an antiproliferative agent to inhibit smooth muscle cell proliferation, i.e., "neointimal hyperplasia," which is believed to be a significant factor leading to re-narrowing or restenosis of the blood vessel after implantation of the stent.

Stents are commonly formed from biocompatible metals such as stainless steel, or metal alloys such as nickel-titanium alloys that are often employed because of their desirable shape-memory characteristics. Other biocompatible metals and metal alloys are used to construct stents. Metallic materials are advantageously employed to construct stents because of the inherent rigidity of metallic materials and the consequent ability of the metallic stent to maintain patency of the lumen upon implantation of the stent.

However, metallic stents are known to cause complications such as thrombosis and neointimal hyperplasia. It is believed that prolonged contact of the metallic surfaces of the stent with the lumen may be a significant factor in these adverse events following implantation. In addition, while metallic stents may provide the rigidity necessary to maintain the patency of the lumen, this rigidity compromises the biomechanical compatibility or compliance of the stent with the lumen walls. This resulting mismatch of compliance between the stent and the lumen walls is also believed to be a factor in neointimal hyperplasia resulting in restenosis.

These adverse events associated with metallic stents can be mitigated somewhat by adapting the stent to provide localized release of a therapeutic agent. In order to provide localized release of a therapeutic agent from a metallic stent, it is known, as described above, to provide the stent with a coating that is adapted to contain therein or thereon one or more therapeutic agents that are released from the coating. Such agents may be incorporated, for example, into a substantially non-biodegradable or biodegradable polymeric material provided as a coating on the metallic stent. In addition to the release of therapeutic agent therefrom, the use of biodegradable polymeric materials as coating layers on metallic stents may be advantageous in initially providing a more biocompatible surface for contact with, for example, the arterial wall. This increased biocompatibility relative to a metallic surface directly contacting the arterial wall may be advantageous in minimizing the likelihood of adverse reactions, such as thrombus formation or restenosis, following implantation.

Biodegradable polymeric materials used to coat metallic stents for providing therapeutic agent delivery are not incorporated within the stent to provide it with mechanical strength necessary for maintaining luminal patency. For example, U.S. Patent No. 6,251,136 B1, incorporated in its entirety herein by reference, discloses at column 1, lines 44-57, that while various polymers are known that are quite capable of carrying and releasing drugs, they generally do not have the requisite strength characteristics. This patent discloses that a previously devised solution to such dilemma has been the coating of a stent's metallic structure with a drug carrying polymer material in order to provide a stent capable of both supporting adequate mechanical loads as well as delivering drugs. Similarly, U.S. Patent No. 5,649,977, incorporated in its entirety herein by reference, discloses at column 4, lines 12-19, a metal reinforced polymer stent wherein the thin metal reinforcement provides the structural strength required for maintaining the patency of the vessel in which the stent is placed, and the polymer coating provides the capacity for carrying and releasing therapeutic drugs at the location of the stent, without significantly increasing the thickness of the stent.

In each of these patents, the metallic component of the coated stent provides the mechanical strength necessary for maintaining the patency of the lumen while the polymeric coating layer functions to deliver therapeutic agent. Because the metallic component provides the structural support, the composite coated stent, while providing beneficial drug delivery, remains relatively rigid and not optimally biomechanically compatible or compliant with the lumen walls. Moreover, in such stents where the coating layer is biodegradable, the coating layer will ultimately be completely biodegraded and or bioresorbed leaving the biomechanically incompatible metallic framework of the stent in direct contact with the lumen walls. The substantial framework of the metallic stent necessary for proper mechanical properties is relatively rigid and not optimally biomechanically compatible or compliant with the lumen walls and also increases the surface area of the metallic structure in contact with the lumen wall. As discussed above, such direct contact of a metallic surface with the lumen walls can result in adverse consequences.

Stents that are completely biodegradable are also known, but there exist distinct disadvantages with such devices that are designed to completely biodegrade *in vivo*. Among such disadvantages include the premature loss of mechanical strength of the device and fragmentation of the device. For example, in the case of an intravascular stent such as a coronary stent commonly used to prevent acute collapse of a coronary vessel after PTCA and to decrease restenosis of the vessel, the loss of mechanical strength can result in the failure of the device to maintain the patency of the coronary vessel during the remodeling and healing period.

It would, therefore, be desirable to provide a stent comprising a composite of metallic and biodegradable polymeric materials wherein the metallic material functions as a reinforcing component but, in the absence of the biodegradable polymeric material, is insufficient to maintain the patency of a lumen upon implantation of the stent. In such a stent, each of the metallic material and the biodegradable polymeric material would cooperate together to provide the mechanical properties necessary for the stent to maintain patency of the lumen upon implantation. In such stent, neither the metallic material nor the biodegradable polymeric material, would act as the substantially sole source of mechanical properties necessary for the stent to maintain patency of the lumen upon implantation.

From WO 01/49335 A1 medical devices consisting of an inorganic substrate and a polymer covering at least a portion of the substrate have become known. The inorganic substrate imparts desired mechanical properties, and the polymer provides appropriate flexibility and/or geometric features.

In WO 01/35859 A1 a stent preform for implantation in a body lumen is described. The preform includes an elongated metal core which is encapsulated in a biocompatible polymer to prevent the core from directly contacting the body lumen.

A covered expanding mesh stent has become known from US 5,674,241. The mesh is metal or plastic. The mesh is covered by an expandable polymer layer, which expands upon hydration and/or without significant resistance.

US 5,443,496 describes a radially expandable stent for implantation within a body lumen having a generally cylindrical body with open proximal and distal ends, the cylindrical body comprising a plurality of metal elements joined to allow flexing of the cylindrical body along the longitudinal axis of the body. The stent exhibits a thin polymeric film extending between the metal elements of the stent.

The problem of the invention is to provide a stent that requires less amounts of metal to increase the biocompatibility, while offering sufficient stability to maintain the patency of a lumen upon implantation of the stent.

### SUMMARY OF THE INVENTION

These and other objects are met by the present invention which provides an intraluminal stent comprising a metallic reinforcing component; and a biodegradable polymeric material covering at least a portion of the metallic reinforcing component. The metallic reinforcing component provides structural reinforcement for the stent, but is insufficient, in the absence of the biodegradable polymeric material, to provide a stent capable of maintaining patency of a lumen upon implantation of the stent into the lumen.

The metallic reinforcing component may be any biocompatible metal. Among preferred biocompatible metals are included those selected from the group consisting of stainless steel, titanium alloys, tantalum alloys, nickel alloys, cobalt alloys and precious metals. Shape memory alloys such as nickel-titanium alloys are particularly preferred. The biodegradable polymeric component may be any biocompatible biodegradable polymer. Among preferred biodegradable polymers are included those selected from the group consisting of polylactic acid, polyglycolic acid, polycaprolactone, polyorthoesters, and trimethylene carbonate polymers, as well as copolymers and mixtures thereof.

The metallic reinforcing component preferably comprises a plurality of apertures or open spaces between metallic filaments, segments or regions. Preferred metallic reinforcing components are selected from the group consisting of an open-mesh network comprising one or more knitted, woven or braided metallic filaments; an interconnected network of articulable segments; a coiled or helical structure comprising one or more metallic filaments; and, a patterned tubular metallic sheet. The metallic reinforcing component may comprise two or more different metals.

In one preferred embodiment, the biodegradable polymeric material is provided as a coating covering at least a portion of the metallic reinforcing component. In other preferred embodiments, the metallic reinforcing component is provided with two or more biodegradable polymeric coating layers. In such embodiments, the biodegradable polymeric coating layers may have different rates of biodegradation. Any one or more of the biodegradable polymeric coating layers may be provided with a therapeutic and/or diagnostic agent therein or thereon. In some preferred embodiments, different therapeutic agents or combinations of therapeutic agents are present in or on two or more of the biodegradable polymeric coating layers.

In another preferred embodiment, the metallic reinforcing component and biodegradable polymeric material are provided within a laminated structure. Preferred laminated structures include those in which the metallic reinforcing component is disposed between two or more layers of biodegradable polymeric material. In some preferred embodiments, the two or more layers of biodegradable polymeric material may comprise different polymeric materials. The two or more layers of biodegradable polymeric material may have different rates of biodegradation. Any one or more of the layers of biodegradable polymeric material comprising the laminated structure may be provided with a therapeutic and/or diagnostic agent therein or thereon. In some preferred embodiments, different therapeutic agents or combinations of therapeutic agents are present in or on two or more of the layers of biodegradable polymeric material.

The intraluminal stent may be any implantable or insertable stent. Such stent may be self-expandable or balloon-expandable. Preferred intraluminal stents are those selected from the group consisting of endovascular, biliary, tracheal, gastrointestinal, urethral, ureteral and esophageal stents. Preferred endovascular stents are coronary stents adapted for implantation into a coronary artery.

One advantage of the present invention is that a stent can be provided with a biodegradable coating that functions to provide structural support and the optional release of a therapeutic agent therefrom.

Another advantage of the present invention is that a stent is provided in which reduced amounts of metallic component remain after degradation of the biodegradable polymeric material covering. As a result, the remaining metallic component is relatively biomechanically compatible or compliant with the lumen walls, and metal-associated complications such as thrombosis and neointimal hyperplasia are minimized.

These and other aspects and advantages of the invention will become apparent from the following detailed description, and the accompanying drawings, which illustrate by way of example the features of the invention.

### BRIEF DESCRIPTION OF THE DRAWINGS

Fig. 1 is a longitudinal perspective view of a metallic reinforcing structure suitable for use in a stent in accordance with the present invention.

Fig. 2 is a partial longitudinal view of a metallic reinforcing structure suitable for use in a stent in accordance with the present invention.

Fig. 3 is a partial longitudinal view of a metallic reinforcing structure suitable for use in a stent in accordance with the present invention.

Fig. 4 is a plan view of a segment of a metallic reinforcing structure suitable for use in the present invention.

Figs. 5a and 5b are longitudinal views of coated metallic filaments suitable for use in forming a stent in accordance with the present invention.

Fig. 6 is a cross sectional end view of the coated metallic filament shown in Fig. 5a.

Fig. 7 is a plan view of a patterned metallic sheet suitable for use in forming a stent in accordance with the present invention.

Fig. 8 is a longitudinal perspective view of a patterned tubular metallic sheet suitable for forming a reinforcing structure for use in a stent in accordance with the present invention.

Fig. 9a is a partial cross-sectional view of a laminated structure suitable for forming a stent in accordance with the present invention.

Fig. 9b is an expanded view of the circled segment of the laminated structure shown in Fig. 9.

It is understood that the above-described Figures are merely simplified schematic representations presented for purposes of illustration only, and the actual structures may differ in numerous respects including the relative scale of the components. The present invention is, therefore, not to be construed as limited to any particular embodiment depicted in these Figures.

### DETAILED DESCRIPTION OF THE INVENTION

The present invention is directed to an intraluminal stent comprising a metallic reinforcing component; and a biodegradable polymeric material covering at least a portion of the metallic reinforcing component. The metallic reinforcing component provides structural reinforcement for the stent but is insufficient, in the absence of the biodegradable polymeric material, to provide a stent capable of maintaining patency of a lumen upon implantation of the stent into the lumen.

The composite intraluminal stent of the present invention, in contrast with known composite stents, utilizes both the metallic component and the biodegradable polymeric component to provide the mechanical properties necessary for maintaining the patency of the lumen upon implantation of the stent into a body lumen. Whereas known composite stents typically employ a biodegradable polymeric component as a coating for incorporating and providing localized release therefrom of a therapeutic agent, such coating layer is not incorporated within the stent to provide it with mechanical strength necessary for maintaining luminal patency. The metallic component, rather than the biodegradable polymeric component, is utilized in such stents to provide the necessary mechanical properties.

While an intraluminal stent in accordance with the present invention can be provided with a drug-releasing biodegradable coating layer, such coating layer, in contrast to other composite stents, cooperates with the metallic component to provide a stent with the requisite mechanical strength to maintain lumen patency. In the absence of the biodegradable polymeric component, the metallic reinforcing component of a stent in accordance with the present invention is insufficient to maintain the patency of the lumen upon implantation.

In the construction of intraluminal stents, metallic materials provide distinct advantages relative to biodegradable polymeric materials and vice versa. For example, metallic materials possess mechanical strength and rigidity whereas biodegradable polymeric materials are often relatively more flexible. The strength of metallic materials is advantageous in constructing intraluminal stents that can maintain lumen patency upon implantation. However, the relative rigidity of metallic materials can be disadvantageous in providing a biomechanically compatible stent that is compliant with the contacting lumen walls. Whereas biodegradable polymeric materials can be more biocompatible and more biomechanically compatible than metallic materials, such materials may not possess the requisite strength to form a stent capable of maintaining lumen patency upon implantation. The present invention provides a composite stent that utilizes both the advantageous strength of metallic materials and the relative biocompatibility and flexibility of biodegradable polymeric materials.

The composite intraluminal stent of the present invention provides distinct advantages relative to composite stents in which the biodegradable polymeric component does not substantially contribute to the mechanical strength of the stent. Because the metallic reinforcing component is not relied on for the sole source of mechanical strength, a stent can be provided that advantageously utilizes less metal and more biodegradable polymeric material. As discussed above, metallic materials are often more rigid and less biocompatible than biodegradable polymeric materials. For example, the relative rigidity of metallic materials can compromise the goal of providing a stent that is biomechanically compatible, i.e., compliant with the contacting lumen walls. Moreover, metallic materials are believed to be associated with complications such as thrombosis and neointimal hyperplasia. This lack of biomechanical compatibility and biocompatiblity can, for example, increase the likelihood of restenosis and other damage to the contacting lumen walls. Because less metal is utilized in a stent in accordance with the present invention, the metallic component of the stent can be constructed from thinner and more flexible metallic filaments or sheets to provide a flexible metallic reinforcing component. Upon in vivo biodegradation of the polymeric material, the remaining flexible metallic framework of the stent will be advantageously less bulky and have a smaller surface area in direct contact with the lumen walls. At such point, the remaining flexible metallic framework of the stent will be compliant with the contacting lumen walls and be less likely to cause damage or injury thereto if left implanted indefinitely.

The metallic reinforcing component may be passivated to inhibit chemical, bio-chemical or electro-chemical interactions with the surrounding blood and tissue to enhance its biostability or biocompatibility within the lumen. Enhanced passivation can be achieved by several methods including the following: formation of stable oxides or nitrides or carbides or mixed compounds on the surface of the metallic reinforcing component. The enhanced passivation can be produced by thermal treatments in controlled atmospheres, physical vapor deposition, chemical vapor deposition, sol gel and electrolytic treatments. Passivated metallic structures suitable for use in the present invention are disclosed in U.S. Patent Application Serial No. 09/815,892, filed March 23, 2001, which is hereby incorporated by reference in its entirety.

By covering at least a portion of the metallic reinforcing component with a biodegradable polymeric material, a composite stent having sufficient mechanical properties to maintain lumen patency upon implantation is, therefore, provided by the present invention. Since both the metallic reinforcing component and the biodegradable polymeric material are relatively flexible, a more biomechanically compatible stent is provided by the present invention. The metallic component reinforces the stent structure, but does not compromise the biomechanical compatibility of the stent as may be the case with a stent that relies solely on a metallic component for mechanical strength. Similarly, a stent constructed solely of biodegradable polymeric materials can prematurely soften or may otherwise not possess the required mechanical strength. In addition, such stents can fragment in vivo and cause localized tissue damage and lumen blockages. By appropriate selection of metallic and biodegradable polymeric materials, the present invention provides an enhanced ability to customize the mechanical properties of an intraluminal stent dependent on the particular application or the time-dependent changes associated with lumen healing or remodeling. The present invention thus relies on the desirable properties of both metallic and biodegradable polymeric materials to provide a composite biomechanically compatible stent.

The metallic reinforcing component of the present invention is preferably an open network comprising a plurality of apertures or open spaces between metallic filaments (including fibers and wires), segments or regions. Preferred metallic reinforcing components are selected from the group consisting of an open-mesh network comprising one or more knitted, woven or braided metallic filaments; an interconnected network of articulable segments; a coiled or helical structure comprising one or more metallic filaments; and, a patterned tubular metallic sheet. Two or more different metals may comprise the metallic reinforcing component. The metallic reinforcing component or a portion thereof can be constructed of a material having a high density, for example platinum, tantalum or gold, to enhance the radio opacity of the composite medical device of the present invention. In general, the metallic reinforcing component can be similar in shape or configuration to any known metallic stent structure, except that the amount of metal is reduced to the point where the metal is insufficient, in the absence of the biodegradable polymeric material, to provide a stent capable of maintaining patency of a lumen upon implantation of the stent into the lumen.

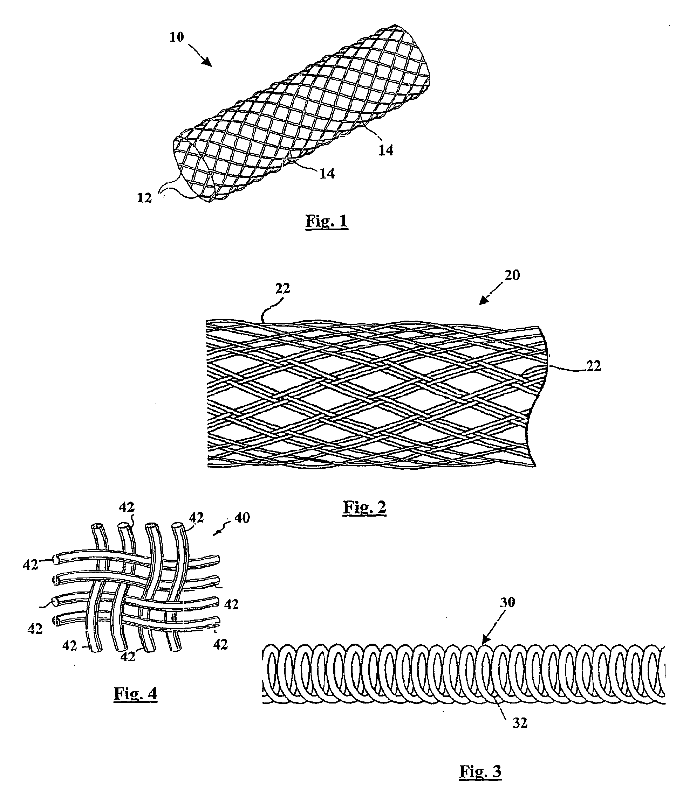

Fig. 1 shows a metallic reinforcing structure 10 suitable for use in a stent in accordance with the present invention. Metallic reinforcing structure 10 is formed from oppositely-directed, parallel, spaced-apart and helically wound elongated strands or filaments 12. The filaments 12 are interwoven and form intersecting points 14 to provide an open mesh or weave construction. Fig. 2 shows a similar metallic reinforcing structure 20, formed from pairs of oppositely-directed, parallel, spaced-apart and helically wound elongated stands or filaments 22. In general, the oppositely-directed helical filaments can comprise, as shown in Fig. 1, one, or as shown in Fig. 2, a plurality of individual metallic filaments. Such metallic filaments may comprise the same or different metals. Fig. 3 shows another metallic reinforcing structure 30 comprising a simple helically coiled metallic strand or filament 32. While Fig. 3 depicts only a single coiled filament, it is understood that more than one filament, of the same or different metals, may be used to form a coiled structure similar to that shown in Fig. 3. Fig. 4 is a generalized depiction of an open mesh network or woven structure 40 that can be used to form a metallic reinforcing component for an intraluminal stent of the present invention. Again, the individual filaments 42 in woven structure 40 may comprise the same or different metals. Similar open-mesh networks comprising knitted or braided filaments can be used to form a metallic reinforcing component for a composite stent of the present invention.

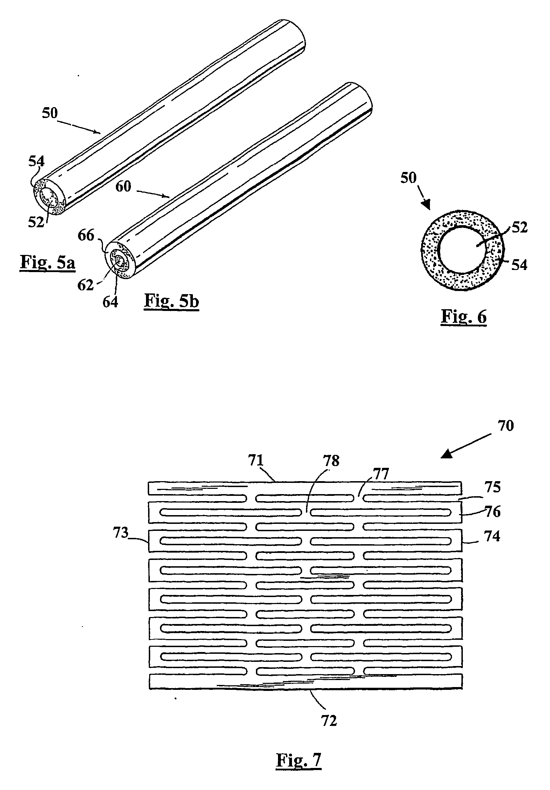

The metallic reinforcing component of the present invention, such as any of those shown in Figs. 1-4, may be a least partially covered with a biodegradable polymeric material to form a biodegradable polymeric material coating layer thereon. The biodegradable polymeric material coating layer may be provided onto individual metallic filaments that are subsequently knitted, woven, braided, coiled or otherwise shaped into an intraluminal stent structure. Alternatively, uncoated filaments may be knitted, woven, braided, coiled or otherwise shaped into a metallic reinforcing structure, which is subsequently coated with a biodegradable polymeric material. Figs. 5a and 5b show coated metallic filaments 50 and 60, respectively, that may form a portion of a composite stent in accordance with the present invention. Coated metallic filament 50 comprises a metallic filament 52 that is coated with a single biodegradable polymeric material coating layer 54. Fig. 6 shows a cross-sectional end view of coated metallic filament 50.

Coated metallic filament 60 of Fig. 5b comprises a metallic filament 62 that is coated with two biodegradable polymeric material coating layers, inner coating layer 64 and outer coating layer 66. It is understood that where multiple coating layers are provided, the layers may comprise different biodegradable polymeric materials and may have different thicknesses. Where two or more biodegradable polymeric material coating layers are provided, it may be advantageous that such coating layers have different rates of biodegradation. For example, in metallic filament 60, outer coating layer 66 may have a faster rate of biodegradation than inner coating layer 64.

A composite stent incorporating multiple layers of biodegradable polymeric material having different rates of biodegradation may be desirable, for example, to effect time-dependent changes in the mechanical properties of the stent as the lumen walls remodel or heal subsequent to implantation of the stent. Further, different rates of biodegradation can be selected to modify the rate of release of any optional therapeutic agent which may be provided in or on any of such multiple coating layers. The incorporation of a therapeutic agent within or on a biodegradable polymeric material utilized in the composite stent of the present invention is discussed more fully below.

Any conventional coating method may be employed to provide a metallic reinforcing component of the present invention with one or more biodegradable polymeric material coating layers. For example, any metallic reinforcing component, such as any metallic filament, metallic segment, patterned metallic sheet or any other metallic region, used in the construction of the stent may be provided with a polymeric material coating layer by dipping the component into a solvent solution or dispersion of the polymer followed by evaporation of the solvent or carrier liquid. A polymer solution or dispersion may also be applied to a metallic reinforcing component by spraying the solution or dispersion onto such component and evaporation of the solvent or carrier liquid. Metallic filaments or sheets may also be provided with one or more coating layers of biodegradable polymeric material by extruding, coextruding or casting a biodegradable polymeric material onto the filament or sheet. Other coating techniques include, for example, coating using fluidized beds or vapor deposition. Coatings may also be formed by in-situ polymerization techniques. It is understood that the present invention is not limited to any particular method of applying a coating layer and, therefore, includes all such methods known to those skilled in the art and adaptable for the purposes described herein.

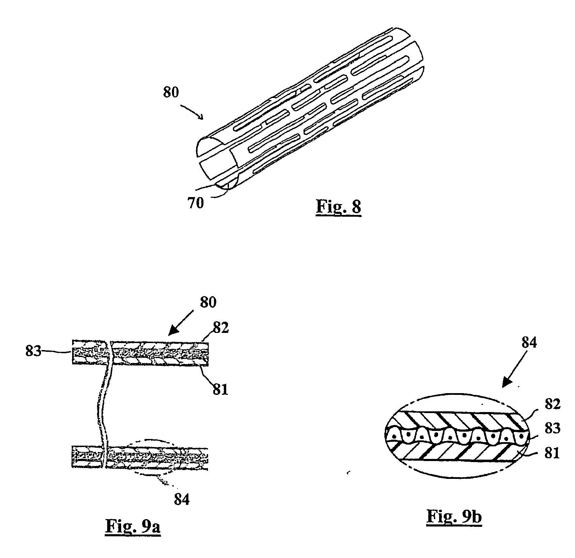

In other embodiments, the metallic reinforcing component of the present invention may comprise a pattered metallic sheet, preferably a pattered tubular metallic sheet. For example, Fig. 7 shows a metallic sheet 70 having a pattern of openings or slots. Metallic sheet 70 comprises top, bottom and sides edges, 71, 72, 73 and 74, respectively; and, rows 75 and 76 of openings or slots. Segments or regions 77 of metallic material between slots in row 75 are staggered with respect to segments or regions 78 of metallic material between slots in adjacent row 76.

With reference to Fig. 8, the patterned metallic sheet 70 is formed into a cylindrical metallic reinforcing member 80 suitable for forming an intraluminal stent in accordance with the present invention. Top and bottom edges 71 and 72 may be attached together by any suitable means such as, for example, by surface fusing, employing plasma energy, laser or ultrasound or with the use of adhesives. Of course, any suitable means for fastening edges 71, 72 together may be employed. The openings or slots in metallic sheet 70 may be formed by any conventional process including, for example, laser cutting or chemical etching of thin metallic sheet stock. It is understood that a patterned metallic sheet for use as a metallic reinforcing component may comprise any pattern of openings or apertures of regular or irregular shape. The openings or apertures need not, of course, extend to the edges of the metallic sheet as shown in Fig. 7.

A patterned metallic sheet may be coated with a biodegradable polymeric material to provide a biodegradable polymeric material coating layer as described above in reference to the coating of knitted, woven, braided or coiled metallic filaments. More than one such biodegradable polymeric material coating layer may be provided, and two or more of such multiple layers may comprise different polymeric materials, have different thicknesses, and/or different rates of biodegradation as discussed above.

Any of the foregoing metallic reinforcing components of the present invention may be provided within a laminated structure comprising two or more layers of biodegradable polymeric material. Fig. 9a is a partial cross-sectional view of a tubular laminated structure 80 useful for forming an intraluminal stent of the present invention. Tubular laminated structure 80 comprises inner and outer layers 81 and 82, respectively, of biodegradable polymeric material with metallic reinforcing component 83 disposed therebetween. Fig. 9b is an expanded view of the circled region 84 shown in Fig. 9a. Any of the two or more layers of biodegradable polymeric material in a laminated structure may comprise the same or different biodegradable polymeric materials and may have different rates of biodegradation.

A laminated structure can be formed by any conventional method of laminating a metallic member between layers of polymeric material. For example, a knitted, braided, woven or coiled metallic reinforcing component or a patterned metallic sheet reinforcing component may be sandwiched between layers of biodegradable polymeric material which may then be fused to the metallic component by the application of heat and/or pressure. Where the metallic reinforcing component is laminated between two layers of the same biodegradable polymeric material, the layers may fuse together between the openings or apertures in the metallic reinforcing component In such case, the biodegradable polymeric material may, in effect, form a single biodegradable polymeric material layer or web between such openings or apertures. In some embodiments of a laminated structure, the biodegradable polymeric material between the openings or apertures defined by the metallic reinforcing member may be completely or partially removed from the resultant laminated structure by, for example, mechanical cutting, laser cutting or dissolving the material with an appropriate solvent. Removal of the polymeric material may employ masking techniques known in the art to protect against removal of biodegradable polymeric layers in contact with the metallic reinforcing component.

As discussed above, the biodegradable polymeric material forming a coating layer or a layer of a laminated structure of a composite stent in accordance with the present invention may be provided therein or thereon with one or more therapeutic agents adapted for localized and/or systemic benefit. Where multiple coating layers or multiple layers of biodegradable polymeric material in a laminated structure are provided, any of such layers, or combination of such layers, may comprise different therapeutic agents or different combinations of therapeutic agents. Where multiple layers each containing one or more therapeutic agents are provided, the layers may be adapted to provide different rates of release of the therapeutic agent or agents incorporated therein or thereon.

The use of different therapeutic agents in different layers, or different rates of release therefrom, may be advantageous, for example, to tailor the spatial and/or temporal release or rate of release of a therapeutic agent from the intraluminal stent. In this manner, the stent may be adapted to provide release of therapeutic agent coincident with the time dependent cellular changes and therapeutic needs at the treatment site and, therefore, increase the efficacy of the therapeutic agent. For example, it may initially be desirable to provide localized release of a therapeutic agent from surfaces of the composite stent in contact with the luminal walls to promote controlled healing and to minimize smooth muscle cell proliferation that can contribute to restenosis. In such case, it may be desirable to provide an initially higher release rate or dosage during the initial stages, for example one to three months after implantation, during which period significant healing and remodeling occurs and the likelihood of restenosis is greater. It may also be desirable to provide inner surfaces of, for example, an endovascular composite stent with antithrombotic therapeutic agent to be released into and therefore minimize the risk of clotting in the blood flowing through the lumen.

"Therapeutic agents", "bioactive agents", "pharmaceutically active agents", "pharmaceutically active materials", "drugs" and other related terms may be used interchangeably herein and include genetic therapeutic agents, non-genetic therapeutic agents and cells.

Exemplary non-genetic therapeutic agents include: (a) anti-thrombotic agents such as heparin, heparin derivatives, urokinase, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anti-coagulants such as D-Phe-Pro-Arg chloromethyl ketone, an RGD peptide-containing compound, heparin, hirudin, antithrombin compounds, platelet receptor antagonists, anti-thrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (1) antimicrobial agents such as triclosan, cephalosporins, aminoglycosides and nitrofurantoin; (m) cytotoxic agents, cytostatic agents and cell proliferation affectors; (n) vasodilating agents; and (o)agents that interfere with endogenous vascoactive mechanisms.

Exemplary genetic therapeutic agents include anti-sense DNA and RNA as well as DNA coding for: (a) anti-sense RNA, (b) tRNA or rRNA to replace defective or deficient endogenous molecules, (c) angiogenic factors including growth factors such as acidic and basic fibroblast growth factors, vascular endothelial growth factor, epidermal growth factor, transforming growth factor α and β, platelet-derived endothelial growth factor, platelet-derived growth factor, tumor necrosis factor α, hepatocyte growth factor and insulin-like growth factor, (d) cell cycle inhibitors including CD inhibitors, and (e) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors of interest for delivery of genetic therapeutic agents include (a) plasmids, (b) viral vectors such as adenovirus, adenoassociated virus and lentivirus, and (c) non-viral vectors such as lipids, liposomes and cationic lipids.

Cells include cells of human origin (autologous or allogeneic), including stem cells, or from an animal source (xenogeneic), which can be genetically engineered if desired to deliver proteins of interest.

A number of the above therapeutic agents and several others have also been identified as candidates for vascular treatment regimens, for example, as agents targeting restenosis. Such agents are appropriate for the practice of the present invention and include one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) ACE inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (1) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, fluvastatin, simvastatin and cerivastatin, (u) fish oils and omega-3-fatty acids, (v) free-radical scavengers/antioxidants such as probucol, vitamins C and E, ebselen, trans-retinoic acid and SOD mimics, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-β pathway agents such as polyanionic agents (heparin, fucoidin), decorin, and TGF-β antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) MMP pathway inhibitors such as marimastat, ilomastat and metastat, (y) cell motility inhibitors such as cytochalasin B, (z) antiproliferative/antineoplastic agents including antimetabolites such as purine analogs(6-mercaptopurine), pyrimidine analogs (e.g., cytarabine and 5-fluorouracil) and methotrexate , nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, paclitaxel and epothilone), caspase activators, proteasome inhibitors, angiogenesis inhibitors (e.g., endostatin, angiostatin and squalamine), rapamycin, cerivastatin, flavopiridol and suramin, (aa) matrix deposition/organization pathway inhibitors such as halofuginone or other quinazolinone derivatives and tranilast, (bb) endothelialization facilitators such as VEGF and RGD peptide, and (cc) blood rheology modulators such as pentoxifylline.

Several of the above and numerous additional therapeutic agents appropriate for the practice of the present invention are also disclosed in U.S. Patent No. 5,733,925 assigned to NeoRx Corporation, the entire disclosure of which is incorporated herein by reference.

The therapeutic agent may be applied onto the device or any portion thereof, for example, by contacting the device or any portion thereof with a solution or suspension of the therapeutic agent, for example by spraying, dipping, and so forth, followed by evaporating the liquid. The drug may also be incorporated during the processing and/or shaping of any of the polymeric materials used to form the medical device of the present invention provided that the drug is stable at the temperature and pressure conditions required during such processing and/or shaping.

Any biodegradable polymeric material forming a coating layer or a layer of a laminated structure of a composite stent in accordance with the present invention may be provided therein or thereon with one or more diagnostic agents such as contrast or radio-opacifying agents to enhance visibility of the device during insertion and subsequent to implantation. Such radio-opacifying agents include, for example, bismuth subcarbonate and others.

The metallic reinforcing component can be any biocompatible metal. Among useful biocompatible metals are included, but are not limited to, stainless steel, titanium alloys, tantalum alloys, nickel alloys such as nickel-chromium alloys, cobalt alloys such as cobalt-chromium alloys and precious metals. Shape memory alloys such as the nickel-titanium alloy, Nitinol® may be used. Shape memory alloys are beneficial, *inter alia,* because they allow the intraluminal stent to be configured in a first condition, i.e., an expanded condition, and then shaped at a different temperature to a second condition, i.e., a smaller condition for loading onto a catheter. The intraluminal stent then regains the memorized enlarged shape when warmed to a selected temperature, such as by exposure to human body temperature or by application of an external heat trigger.

The biodegradable polymeric material utilized in the composite stent of the present invention may be any biocompatible biodegradable, bioresorbable or bioerodable polymeric material. Any portion of an intraluminal stent or other medical device described herein as "biodegradable," "bioresorbable," or "bioerodable" will, over time, lose bulk mass by being degraded, resorbed or eroded by normal biological processes in the body. As used herein, the term "biodegradable" is intended to encompass the terms "bioresorbable" and "bioerodable." Typically, the material is metabolized or broken down by normal biological processes into metabolites or break-down products that are substantially non-toxic to the body and are capable of being resorbed and/or eliminated through normal excretory and metabolic processes of the body. Such biological processes include those that are primarily mediated by metabolic routes such as enzymatic action or by simple hydrolytic action under normal physiological pH conditions.

The biodegradable polymeric material utilized in the present invention may be either a "surface erodable" or a "bulk erodable" biodegradable material. Or a biodegradable material that is both surface and bulk erodable. Surface erodable materials are materials in which bulk mass is lost primarily at the surface of the material that is in direct contact with the physiologic environment, such as body fluids. Bulk erodable materials are materials in which bulk mass is lost throughout the mass of the material, i.e., loss of bulk mass is not limited to mass loss that occurs primarily at the surface of the material in direct contact with the physiological environment.

Among biodegradable polymeric materials that can be utilized in the present invention are included, but not limited to, poly(L-lactide) (PLLA), poly(D,L-lactide) (PLA), polyglycolide (PGA), poly(L-lactide-co-D,L-lactide) (PLLA/PLA), poly(L-lactide-co-glycolide) (PLLA/PGA), poly(D, L-lactide-co-glycolide) (PLA/PGA), poly(glycolide-co-trimethylene carbonate) (PGA/PTMC), polyethylene oxide (PEO), polydioxanone (PDS), polypropylene fumarate, poly(ethyl glutamate-co-glutamic acid), poly(tert-butyloxy-carbonylmethyl glutamate), polycaprolactone (PCL), polycaprolactone co-butylacrylate, polyhydroxybutyrate (PHBT) and copolymers of polyhydroxybutyrate, poly(phosphazene), poly(D,L-lactide-co-caprolactone) (PLA/PCL), poly(glycolide-co-caprolactone) (PGA/PCL), poly(phosphate ester), polyamides, polyorthoesters and polyanhydrides (PAN), maleic anhydride copolymers, and polyhydroxybutyrate copolymers, poly(amino acid) and poly(hydroxy butyrate), polydepsipeptides, maleic anhydride copolymers, polyphosphazenes, polyiminocarbonates, poly[(97.5% dimethyltrimethylene carbonate)-co-(2.5% trimethylene carbonate)], cyanoacrylate, polyethylene oxide, hydroxypropylmethylcellulose, polysaccharides such as hyaluronic acid, chitosan and regenerate cellulose, and proteins such as gelatin and collagen, among others. Preferred biodegradable polymeric materials are selected from the group consisting of polylactic acid, polyglycolic acid, polycaprolactone, polyorthoesters, and trimethylene carbonate polymers, as well as copolymers and mixtures thereof.

The biodegradable polymeric material may be a biodegradable shape memory material. Biodegradable shape memory materials are disclosed, e.g., in U.S. Patent No. 6,160,084, the entirety of which is herein incorporated by reference. Such materials function similarly to shape memory metallic alloys such as Nitinol® by "remembering" their initial shape. The memory can be triggered by the application of heat to the material configured to a different shape. Thus, when a shape memory polymer is heated above the melting point or glass transition temperature of hard segments in the polymer backbone, the material can be shaped. This (original) shape can be memorized by cooling the shape memory polymer below the melting point or glass transition temperature of the hard segment. When the shaped shape memory polymer is cooled below the melting point or glass transition temperature of a soft segment in the polymer backbone, while the shape is deformed, a new (temporary) shape is fixed. The original shape is recovered by heating the material above the melting point or glass transition temperature of the soft segment but below the melting point or glass transition temperature of the hard segment.

The use of biodegradable shape memory polymers, as with the use of shape memory alloys, is advantageous in that a medical device constructed of such material can be mounted onto a delivery device such as a catheter in compressed shape, and be triggered to return to its memory shape by, e.g., raising its temperature above the transition temperature. This could be accomplished, for example, by contact with body temperature or application of an external heat trigger. It may be preferable that where a shape memory alloy such as Nitinol is used to form the metallic reinforcing component, the biodegradable polymeric material is a shape memory biodegradable polymer.

Shape memory biodegradable polymers whose shape change is triggered optically by, for example, application of light to the material are also useful biodegradable materials in the medical devices of the present invention.

The present invention may be adapted to be utilized with any implantable or insertable medical device that may beneficially be constructed from a composite of metallic and biodegradable polymeric materials. Thus, the present invention has broad application to any medical device, such as those typically constructed of metallic materials, by providing a composite medical device in which the metallic component, in the absence of the biodegradable polymeric material, would not possess the mechanical strength required for proper functioning of the device. Implantable or insertable medical devices with the scope of the present invention, therefore, include, but are not limited to, stents of any shape or configuration, stent grafts, catheters, cerebral aneurysm filler coils, vascular grafts, vena cava filters, heart valve scaffolds and other implantable or insertable medical devices. However, intraluminal stents such as endovascular, biliary, tracheal, gastrointestinal, urethral, ureteral, and esophageal stents are preferred composite medical devices of the present invention. Particularly preferred intraluminal stents are coronary vascular stents. The composite intraluminal stents of the present invention may be balloon-expandable or self-expandable.

While the invention described hereinabove has been particularly shown and described with reference to specific embodiments thereof, the invention is not to be limited by the described embodiments and any accompanying Figures. The spirit and scope of the invention is, therefore, indicated only by the appended claims. All changes that come within the meaning and range of equivalents of the appended claims are intended be encompassed within the scope thereof.

## Claims

1. An intraluminal stent comprising:
a metallic reinforcing component; and
a biodegradable polymeric material;
**characterized in that** the biodegradable polymeric material covers at least a portion of the metallic reinforcing component and **in that** the metallic reinforcing component provides structural reinforcement for the stent but is insufficient, in the absence of the biodegradable polymeric material, to provide a stent capable of maintaining patency of a lumen upon implantation of the stent into the lumen.

2. The intraluminal stent of claim 1, wherein the metallic reinforcing component comprises a biocompatible metal selected from the group consisting of stainless steel, titanium alloys, tantalum alloys, nickel alloys, cobalt alloys and precious metals.

3. The intraluminal stent of claim 2, wherein the biocompatible metal comprises a shape memory alloy.

4. The intraluminal stent of claim 3, wherein the shape memory alloy comprises a nickel-titanium alloy.

5. The intraluminal stent of one of the claims 1 to 4, wherein the biodegradable polymeric material comprises a biocompatible biodegradable polymer selected from the group consisting of polyactic acid, polyglycolic acid, polycaprolactone, polyorthoesters and trimethylene carbonate polymers, as well as copolymers and mixtures thereof.

6. The intraluminal stent of one of the claims 1 to 5, wherein the stent is selected from the group consisting of endovascular, biliary, tracheal, gastrointestinal, urethral, ureteral and esophageal stents.

7. The intraluminal stent of claim 6, wherein the stent is balloon-expandable or self-expandable.

8. The intraluminal stent of claim 6 or 7, wherein the endovascular stent is a coronary stent.

9. The intraluminal stent of claim 1, wherein the metallic reinforcing component comprises a plurality of apertures.

10. The intraluminal stent of claim 9, wherein the metallic reinforcing component is selected from the group consisting of an open-mesh network comprising one or more knitted, woven or braided metallic filaments; an interconnected network of articulable segments, a coiled or helical structure comprising one or more metallic filaments; and, a patterned tubular metallic sheet.

11. The intraluminal stent of claim 10, wherein said metallic filaments comprise two or more different metals.

12. The intraluminal stent of claim 10, wherein the patterned tubular metallic sheet is formed by laser-cutting or chemical etching of a metallic sheet.

13. The intraluminal stent of one of the claims 9 to 12, wherein the biodegradable polymeric material covering at least a portion of the metallic reinforcing component comprises one, two or more biodegradable polymeric material coating layer(s).

14. The intraluminal stent of claim 13, wherein said biodegradable polymeric material coating layer(s) comprise(s) one or more therapeutic and/or diagnostic agents.

15. The intraluminal stent of claim 14, wherein different therapeutic agents or combinations of therapeutic agents are present in two of more of said biodegradable polymeric material coating layers.

16. The intraluminal stent of claim 13, wherein at least two of said biodegradable polymeric material coating layers have different rates of biodegradation.

17. The intraluminal stent of claim 14, wherein at least two of said biodegradable polymeric material coating layers have different rates of release of therapeutic agent therefrom.

18. The intraluminal stent of one of the claims 10 to 17, wherein the metallic reinforcing component and biodegradable polymeric material are provided within a laminated structure.

19. The intraluminal stent of claim 18, wherein the metallic reinforcing component is disposed between two or more layers of the biodegradable polymeric material.

20. The intraluminal stent of claim 19, wherein the two or more layers comprise different biodegradable polymeric materials.

21. The intraluminal stent of claim 19, wherein at least one of said two or more layers comprises one or more therapeutic and/or diagnostic agents.

22. The intraluminal stent of claim 21, wherein different therapeutic agents or combinations of therapeutic agents are present in two or more of said layers.

23. The intraluminal stent of one of the claims 19 to 22, wherein at least two of said layers have different rates of biodegradation.

24. The intraluminal stent of claim 21, wherein at least two of said layers have different rates of release of therapeutic agent therefrom.

25. The intraluminal stent of one of the claims 1 to 24, wherein a surface of the metallic reinforcing component is passivated to enhance its biocompatibility.

## Patentansprüche

1. Ein intraluminaler Stent, der umfasst:
eine metallische verstärkende Komponente und
ein biologisch abbaubares polymeres Material;
**dadurch** charakterisiert, dass
das biologisch abbaubare polymere Material zumindest einen Teil der metallischen verstärkenden Komponente bedeckt, und
die metallische verstärkende Komponente dem Stent strukturelle Verstärkung liefert, aber, bei Abwesenheit des biologisch abbaubaren polymeren Materials, nicht ausreicht, um einen Stent zu liefern, der in der Lage ist, die Durchgängigkeit eines Hohlraums nach der Implantation des Stents in den Hohlraum aufrechtzuerhalten.

2. Der intraluminale Stent gemäß Anspruch 1, wobei die metallische verstärkende Komponente ein biologisch verträgliches Metall umfasst, das aus einer Gruppe ausgewählt wird, die aus rostfreiem Stahl, Titanlegierungen, Tantallegierungen, Nickellegierungen, Kobaltlegierungen und Edelmetallen besteht.

3. Der intraluminale Stent gemäß Anspruch 2, wobei das biologisch verträgliche Metall eine formspeichernde Legierung aufweist.

4. Der intraluminale Stent gemäß Anspruch 3, wobei die formspeichernde Legierung eine Nickel-Titan Legierung umfasst.

5. Der intraluminale Stent gemäß einem der Ansprüche 1 bis 4, wobei das biologisch abbaubare polymere Material ein biologisch verträgliches, biologisch abbaubares Polymer umfasst, das aus der Gruppe ausgewählt wird, die aus polyaktischer Säure, Polyglykolsäure, Polykaprolakton, Polyorthoester und Trimethylenkarbonat Polymeren, ebenso wie aus Copolymeren und Mischungen davon, besteht.

6. Der intraluminale Stent gemäß einem der Ansprüche 1 bis 5, wobei der Stent aus einer Gruppe ausgewählt wird, die aus endovaskulären, die Gallenwege, die Luftröhre, den Magen und den Darm, die Harnröhre, den Harnleiter und die Speiseröhre betreffenden Stents besteht.

7. Der intraluminale Stent gemäß Anspruch 6, wobei der Stent ballonexpandierbar oder selbstexpandierbar ist.

8. Der intraluminale Stent gemäß den Ansprüchen 6 oder 7, wobei der endovaskuläre Stent ein koronarer Stent ist.

9. Der intraluminale Stent gemäß Anspruch 1, wobei die metallische verstärkende Komponente eine Vielzahl von Öffnungen aufweist.

10. Der intraluminale Stent gemäß Anspruch 9, wobei die metallische verstärkende Komponente aus einer Gruppe ausgewählt wird, die aus einem Netzwerk mit offenen Maschen, das eine oder mehrere gestrickte, geflochtene oder umsponnene metallische Fäden umfasst; aus einem verbundenen Netzwerk von gegliederten Segmenten; einer gerollten oder spiralförmige Struktur, die einen metallischen Faden oder mehrere metallische Fäden aufweist; und einer gemusterten rohrförmigen metallischen Folie besteht.

11. Der intraluminale Stent gemäß Anspruch 10, wobei die besagten metallischen Fäden zwei oder mehrere verschiedene Metalle umfassen.

12. Der intraluminale Stent gemäß Anspruch 10, wobei die gemusterte rohrförmige metallische Folie durch Laserschnitt oder chemisches Ätzen einer metallischen Folie geformt wird.

13. Der intraluminale Stent gemäß einem der Ansprüche 9 bis 12, wobei das biologisch abbaubare polymere Material, das zumindest einen Teil der metallischen verstärkenden Komponente bedeckt, eine, zwei oder mehrere überziehende Schicht(en) aus biologisch abbaubarem polymerem Material umfasst.

14. Der intraluminale Stent gemäß Anspruch 13, wobei besagte überziehende Schicht(en) aus biologisch abbaubarem polymerem Material einen oder mehrere therapeutische und/oder diagnostische Wirkstoffe umfasst (umfassen).

15. Der intraluminale Stent gemäß Anspruch 14, wobei verschiedene therapeutische Wirkstoffe oder Kombinationen therapeutischer Wirkstoffe in zwei von mehreren besagter überziehender Schichten aus biologisch abbaubarem polymerem Material vorhanden sind.

16. Der intraluminale Stent gemäß Anspruch 13, wobei zumindest zwei der besagten überziehenden Schichten aus biologisch abbaubarem polymerem Material unterschiedliche Grade von biologischem Abbau aufweisen.

17. Der intraluminale Stent gemäß Anspruch 14, wobei zumindest zwei der besagten überziehenden Schichten aus biologisch abbaubarem polymerem Material in unterschiedlichen Maßen therapeutischen Wirkstoff von dort freisetzen.

18. Der intraluminale Stent gemäß einem der Ansprüche 10 bis 17, wobei die metallische verstärkende Komponente und das biologisch abbaubare polymere Material in einer mehrschichtigen Struktur bereitgestellt werden.

19. Der intraluminale Stent gemäß Anspruch 18, wobei die metallische verstärkende Komponente zwischen zwei oder mehreren Schichten des biologisch abbaubaren polymeren Materials angeordnet ist.

20. Der intraluminale Stent gemäß Anspruch 19, wobei die zwei oder mehreren Schichten verschiedene biologisch abbaubare polymere Materialien umfassen.

21. Der intraluminale Stent gemäß Anspruch 19, wobei zumindest eine der besagten zwei oder mehreren Schichten einen oder mehrere therapeutische und/oder diagnostische Wirkstoffe umfasst.

22. Der intraluminale Stent gemäß Anspruch 21, wobei verschiedene therapeutische Wirkstoffe oder Kombinationen therapeutischer Wirkstoffe in zwei oder mehreren der besagten Schichten vorhanden sind.

23. Der intraluminale Stent gemäß einem der Ansprüche 19 bis 22, wobei zumindest zwei der besagten Schichten unterschiedliche Grade von biologischem Abbau aufweisen.

24. Der intraluminale Stent gemäß Anspruch 21, wobei zumindest zwei der besagten Schichten verschiedene Grade der Freisetzung des therapeutischen Wirkstoffs besitzen.

25. Der intraluminale Stent gemäß einem der Ansprüche 1 bis 24, wobei eine Oberfläche der metallischen verstärkenden Komponente passiviert ist, um seine Biokompatibilität zu verbessern.

## Revendications

1. Extenseur endoluminal comprenant :
un composant de renfort métallique ; et
un matériau polymère biodégradable ;
**caractérisé en ce que** le matériau polymère biodégradable couvre au moins une partie du composant de renfort métallique et **en ce que** le composant de renfort métallique fournit un renfort structurel à l'extenseur qui est insuffisant, en l'absence du matériau polymère biodégradable, pour fournir un extenseur capable de maintenir la perméabilité d'une lumière lors de l'implantation de l'extenseur dans la lumière.

2. Extenseur endoluminal selon la revendication 1, dans lequel le composant de renfort métallique comprend un métal biocompatible sélectionné dans le groupe constitué de l'acier inoxydable, des alliages de titane, des alliages de tantale, des alliages de nickel, des alliages de cobalt et des métaux précieux.

3. Extenseur endoluminal selon la revendication 2, dans lequel le métal biocompatible est constitué d'un alliage à mémoire de forme.

4. Extenseur endoluminal selon la revendication 3, dans lequel l'alliage à mémoire de forme comprend un alliage nickel-titane.

5. Extenseur endoluminal selon l'une quelconque des revendications 1 à 4, dans lequel le matériau polymère biodégradable est constitué d'un polymère biodégradable biocompatible sélectionné dans le groupe constitué par l'acide polylactique, l'acide polyglycolique, le polycaprolactone, les polyorthoesters et les polymères de carbonate de triméthylène, ainsi que les copolymères et mélanges de ceux-ci.

6. Extenseur endoluminal selon l'une quelconque des revendications 1 à 5, dans lequel l'extenseur est sélectionné dans le groupe constitué par les extenseurs endovasculaires, biliaires, trachéaux, gastro-intestinaux, urétraux, urétéraux et oesophagiques.

7. Extenseur endoluminal selon la revendication 6, dans lequel l'extenseur est expansible par ballon ou auto-expansible.

8. Extenseur endoluminal selon la revendication 6 ou 7, dans lequel l'extenseur endoluminal est un extenseur coronarien.

9. Extenseur endoluminal selon la revendication 1, dans lequel le composant de renfort métallique comprend une pluralité d'ouvertures.

10. Extenseur endoluminal selon la revendication 9, dans lequel le composant de renfort métallique est sélectionné dans le groupe constitué d'un réseau de maillage ouvert constitué d'un ou plusieurs filaments métalliques tricotés, tissés ou tressés ; d'un réseau interconnecté de segments articulables, d'une structure enroulée ou hélicoïdale constituée d'un ou plusieurs filaments métalliques ; et d'une feuille métallique tubulaire à motifs.

11. Extenseur endoluminal selon la revendication 10, dans lequel lesdits filaments métalliques sont constitués de deux métaux différents ou plus.

12. Extenseur endoluminal selon la revendication 10, dans lequel la feuille métallique tubulaire à motifs est formée par découpage au laser ou par attaque chimique d'une feuille métallique.

13. Extenseur endoluminal selon l'une quelconque des revendications 9 à 12, dans lequel le matériau polymère biodégradable couvrant au moins une partie du composant de renfort métallique comprend une, deux ou plus de deux couche(s) de revêtement de matériau polymère biodégradable.

14. Extenseur endoluminal selon la revendication 13, dans lequel ladite ou lesdites couche(s) de revêtement de matériau polymère biodégradable comprend ou comprennent un ou plusieurs agent(s) thérapeutique(s) et / ou diagnostique(s).

15. Extenseur endoluminal selon la revendication 14, dans lequel différents agents thérapeutiques ou combinaisons d'agents thérapeutiques sont présents dans deux ou plus de deux desdites couches de revêtement de matériau polymère biodégradable.

16. Extenseur endoluminal selon la revendication 13, dans lequel au moins deux desdites couches de revêtement de matériau polymère biodégradable ont des vitesses de biodégradation différentes.

17. Extenseur endoluminal selon la revendication 14, dans lequel au moins deux desdites couches de revêtement de matériau polymère biodégradable ont des vitesses différentes de libération de l'agent thérapeutique.

18. Extenseur endoluminal selon l'une quelconque des revendications 10 à 17, dans lequel le composant de renfort métallique et le matériau polymère biodégradable sont fournis dans une structure stratifiée.

19. Extenseur endoluminal selon la revendication 18, dans lequel le composant de renfort métallique est disposé entre deux couches de matériau polymère biodégradable ou plus.

20. Extenseur endoluminal selon la revendication 19, dans lequel les deux couches ou plus sont constituées de matériaux polymères biodégradables différents.

21. Extenseur endoluminal selon la revendication 19, dans lequel au moins une desdites deux couches ou plus comprend un ou plusieurs agent(s) thérapeutique(s) et / ou diagnostique(s).

22. Extenseur endoluminal selon la revendication 21, dans lequel des agents thérapeutiques ou combinaisons d'agents thérapeutiques différents sont présents dans deux desdites couches ou plus.

23. Extenseur endoluminal selon l'une des revendications 19 à 22, dans lequel au moins deux desdites couches ont des vitesses de biodégradation différentes.

24. Extenseur endoluminal selon la revendication 21, dans lequel au moins deux desdites couches ont des vitesses de libération de l'agent thérapeutique différentes.

25. Extenseur endoluminal selon l'une des revendications 1 à 24, dans lequel une surface du composant de renfort métallique est passivée pour en améliorer la biocompatibilité.
